# EUROPEAN PATENT SPECIFICATION

(11) **EP 3 641 747 B1**
(45) Date of publication and mention of the grant of the patent: **06.09.2023**
(21) Application number: 18746305.4
(22) Date of filing: 22.06.2018
(51) Int. Cl.: A61K 31/192, A61K 31/35, A61K 31/4184, A61K 31/4375, A61K 31/47, A61K 31/4709, A61K 31/473, A61K 31/496, A61K 31/506, A61K 31/513, A61K 31/519, A61K 31/5383, A61K 45/06, A61K 9/00, A61P 35/00, A61P 35/02, A61P 35/04

(54) **PAR-4 SECRETAGOGUES FOR THE TREATMENT OF CANCER**
SECRETAGOGUE FÜR PAR-4 ZUR BEHANDLUNG VON KREBS
SÉCRETAGOGUES DE PAR-4 DESTINÉES AU TRAITEMENT DU CANCER

(30) Priority: 22.06.2017 IN 201721021945
(43) Date of publication of application: 29.04.2020
(73) Proprietor: Cipla Limited, Mumbai, Maharashtra 400013 (IN)
(72) Inventor: MALHOTRA, Geena, Mumbai Maharashtra 400014 (IN); JOSHI, Kalpana, Thane Maharashtra 400605 (IN); GHOSALKAR, Jeevan, Thane (West) Maharashtra 400601 (IN)
(74) Representative: Paulraj, Leonita Theresa
(86) International application number: PCT/IN2018/050409
(87) International publication number: WO 2018/235103

(56) References cited:
- WO-A1-2015/077550
- WO-A1-2016/196614
- US-A1- 2016 332 971
- US-A1- 2017 165 257
- US-A1- 2017 368 072
- MILLER S C ET AL: "Identification of known drugs that act as inhibitors of NF-@?B signaling and their mechanism of action", BIOCHEMICAL PHARMACOLOGY, ELSEVIER, US, vol. 79, no. 9, 1 May 2010 (2010-05-01), pages 1272-1280, XP026939405, ISSN: 0006-2952, DOI: 10.1016/J.BCP.2009.12.021 [retrieved on 2010-03-06] cited in the application

## Description

### FIELD OF INVENTION:

The present invention relates to a prostate apoptosis response-4 (PAR-4) inducing agent for use in the treatment of cancer in a subject in need thereof. The PAR-4 inducing agent (generally referred as secretagogues) is administered in amounts sufficient to induce production and/or secretion of the tumor suppressor PAR-4 by cells, preferably in an amount sufficient to inhibit proliferation and/or metastasis of cancer cells, and/or reduce the recurrence of tumors. The PAR-4 inducing agent may be administered with or without a chemotherapeutic and other anticancer therapy. The PAR-4 inducing agent may also optionally be administered with ionizing radiation.

### BACKGROUND OF INVENTION:

The development and progression of cancer is a multistep process involving accumulation of multiple genetic aberrations. Most notable among such aberrations is the loss of apoptotic responses that normally serve as built in checkpoints against the emergence of cell populations with dysfunctional traits or the acquisition of prosurvival mechanisms that override the apoptotic signals. The loss of apoptotic mechanisms often results in abridged response to cancer therapy. As such, alternate or combinatorial approaches that kill cancer cells and induce tumor regression are being actively pursued by researchers and physicians.

Especially difficult to treat are those cancers which are hormonally related and/or are metastatic cancers. These cancers include, e.g., prostate cancer, breast cancer and lung cancer. Melanoma is also difficult to treat and has a low survival rate relative to many other cancers.

An essential feature of anticancer strategies is the selective action against cancer cells, with little or no damage inflicted in normal cells. Nonetheless, side effects of cancer therapies are often severe. They include nausea, vomiting, pain, poor appetite, wasting, cachexia, diarrhea, burning in the stomach, stress, planter warts, nerve death-neuropathy, radiation burns, fatigue, constipation, anemia, anxiety, weakened immune system, dry skin, bone marrow suppression and hair loss. As such the identification of molecules that can specifically target tumor cells, with minimal or no adverse effects to normal cells constitutes a significant area of cancer research. Such molecules with selective action against tumor cells are valuable not only for their therapeutic potential; but also for their potential applications as tools for dissection of fundamental differences between normal and cancer cells. Thus, treatment methods that specifically target certain types of hormonally linked cancers would be extremely useful. Additionally, treatment methods that target cancers located in highly vascularized tissues such as for example lung, kidney, liver, or blood, and methods that target difficult to treat cancers, such as for example melanoma, would also be highly beneficial.

WO 2016/196614, US 2016/332971 and WO 2015/077550 disclose PAR-4 secretagogues for use in the treatment of cancer.

### OBJECT OF THE INVENTION:

An object of the present invention is to provide PAR-4 inducing agents for use in a method of treating cancer.

Another object of present invention is to provide agents for use in a method of treating cancer in a population of cells comprising the cancer cell and normal cells.

Another object of present invention is to provide agents for use in a method of treating cancer in a population of cells comprising the cancer cell and normal cells with an effective amount of agent or pharmaceutically acceptable derivative thereof for a sufficient time.

Also disclosed is a pharmaceutical composition comprising PAR-4 inducing agent for the treatment of cancer.

Also disclosed is the use of a PAR-4 inducing agent for inducing PAR-4 in cell.

Also disclosed is the use of a PAR-4 inducing agent for inducing GRP-78 in cell.

### SUMMARY OF THE INVENTION:

In its broadest sense, the present invention provides an agent for use in the treatment of cancer by contacting a population of cells comprising cancer cells and normal cells with an effective amount of the agent which is selected from a group consisting of: mebendazole, pyronaridine, tafenoquine, minoxidil, nalidixic acid, sparfloxacin, pipemidic acid and ofloxacin.

Also disclosed is a pharmaceutical composition comprising a PAR-4 inducing agent for the treatment of cancer.

Also disclosed is a method of inducing PAR-4 comprising administrating PAR-4 inducing agent.

Also disclosed is the use of a PAR-4 inducing agent for inducing GRP-78 in cell.

### BRIEF DESCRIPTION OF THE DRAWINGS:

Figure 1: Dose dependent induction of PAR-4 by Pyronaridine in Mouse embryonic fibroblast cells
Figure 2: Dose dependent induction of PAR-4 by Terconazole in cell supernatant of Mouse embryonic fibroblast cells
Figure 3: Induction of PAR-4 by Terconazole in cell lysate of Mouse embryonic fibroblast cells
Figure 4: Induction of PAR-4 by Mefloquine in cell supernatant of Mouse embryonic fibroblast cells
Figure 5: Relative to vehicle treatment, Mefloqiune induced robust elevation of PAR-4 in mouse at 51.2 mg/kg.
Figure 6: Induction of GRP-78 by Mefloquine in ovarian and renal cancer cell lines
Figure 7: Induction of PAR-4 by Narasin in cell supernatant of Mouse embryonic fibroblast cells
Figure 8: Relative to vehicle treatment, Narasin induced robust elevation of PAR-4 in mouse at 1.5 mg/kg
Figure 9: Induction of PAR-4 by Mebendazole in cell supernatant and lysate of Mouse embryonic fibroblast cells
Figure 10: Induction of GRP-78 by Mebendazole in ovarian and renal cancer cell lines
Figure 11: Induction of PAR-4 by Tafenoquine in cell supernatant and lysate of Mouse embryonic fibroblast cells
Figure 12: Dose dependent induction of PAR-4 by Tafenoquine in cell supernatant and lysate of Mouse embryonic fibroblast cells
Figure 13: Induction of GRP-78 by Tafenoquine in ovarian and renal cancer cell lines.
Figure 14: Induction of PAR-4 by Minoxidil in cell supernatant of Mouse embryonic fibroblast cells.
Figure 15: Induction of PAR-4 by Nalidixic Acid in cell supernatant of Mouse embryonic fibroblast cells.
Figure 16: Induction of PAR-4 by sparfloxacin in cell supernatant of Mouse embryonic fibroblast cells.
Figure 17: Induction of PAR-4 by Pipemidic Acid in cell supernatant of Mouse embryonic fibroblast cells.
Figure 18: Induction of PAR-4 by Lopinavir in cell supernatant of Mouse embryonic fibroblast cells.
Figure 19: Induction of PAR-4 by Ofloxacin in cell supernatant of Mouse embryonic fibroblast cells.

### DETAILED DESCRIPTION OF THE INVENTION:

The tumor suppressor PAR-4 (prostate apoptosis response-4) induces apoptotic cell death specifically in cancer cells but not in normal cells. This cancer-selective action is attributed to its centrally located SAC domain (EI-Guendy et al. (2003) "Identification of a unique core domain of PAR-4 sufficient for selective apoptosis-induction in cancer cells." Mol. Cell. Biol. 23, 5516-5525).

The PAR-4 protein has not only an intracellular function, but it is also secreted by both normal and cancer cells (Burikhanov et al. (2009). "The tumor suppressor PAR-4 activates an extrinsic pathway for apoptosis" Cell 138, 377-388.). Secreted PAR-4 binds to its receptor GRP-78, which is upregulated on the surface of cancer cells, and induces apoptosis (Burikhanov et al. (2009); Bhattarai, T, and Rangnekar VM (2010) "Cancer- selective apoptotic effects of extracellular and intracellular PAR-4" Oncogene 29, 3873-3880). The basal level of PAR-4 secreted by normal cells is inadequate to induce apoptosis of cancer cells or inhibit the growth of tumors (Burikhanov et al. (2009)). However, elevated levels of extracellular PAR-4 produced by injecting recombinant PAR-4 in mice, cause inhibition of metastatic lung tumors (Zhao et al. (2011) "Systemic PAR-4 inhibits metastatic tumor growth". Cancer Biol Ther. 12, 152-157). [0008] There are several non-FDA approved small molecules, such as Nutlin-3a, PS- 1145, and Arylquin- 1 (INV13/1947) that can increase secretion of PAR-4 from normal cells in mice and the sera from these mice induced ex vivo apoptosis in cancer cell cultures (Burikhanov et al. (2014) "Paracrine apoptotic effect of p53 mediated by tumor suppressor PAR-4". Cell Reports 6, 271-277; and Burikhanov et al., "Arylquin- 1 targets vimentin to trigger PAR-4 secretion for tumor cell apoptosis" Nature Chem Biol. 10, 924-926 (2014)).

PAR-4 inducing agents are those molecules which induce the secretion of PAR-4 from normal cells which induces apoptotic cell death specifically in cancer cells but not in normal cells.

According to the present invention, the PAR-4 inducing agents are Mebendazole, Pyronaridine, Tafenoquine, Minoxidil, Nalidixic Acid, Sparfloxacin, Pipemidic Acid, and Ofloxacin.

Surprisingly, the inventors have found that Adapalene, Narasin, Mefloquine, Mebendazole, Terconazole, Pyronaridine, Tafenoquine, Minoxidil, Nalidixic Acid, Sparfloxacin, Pipemidic Acid, Lopinavir and Ofloxacin possess PAR-4 inducing activity. In particular, described herein is the surprising effect of Adapalene, Narasin, Mefloquine, Mebendazole, Terconazole, Pyronaridine, Tafenoquine, Minoxidil, Nalidixic Acid, Sparfloxacin, Pipemidic Acid, Lopinavir and Ofloxacin including their various salt forms, to induce robust PAR-4 production from human and mouse cells and can be used to inhibit proliferation and/or metastasis of cancer cells, and to inhibit recurrent tumor formation in subjects in need thereof.

Described herein are methods for harnessing the ability of PAR-4 secreted from cells to induce apoptosis and inhibit tumor growth of cancer cells. In the methods described herein, also an aspect of this invention is a method for treating a subject having cancer cells that are p53-deficient but nonetheless unexpectedly undergo apoptosis in response to PAR-4 exposure, by administering an effective amount of PAR-4 inducing agent to the subject. An effective amount of PAR-4 inducing agent is sufficient to increase PAR-4 secretion by cells to a level that induces apoptosis of the cancer cells, and/or inhibits proliferation of such cancer cells and/or inhibits metastasis of such cancer cells. In an embodiment, the agent for use of present invention comprises contacting a population of cells comprising cancer cells and normal cells with an effective amount of PAR-4 inducing agents or pharmaceutically acceptable derivative thereof, wherein treatment with the effective amount of PAR-4 inducing agents or pharmaceutically acceptable derivative thereof, kills cancer cells, inhibits cancer cell proliferation, cancer cell metastasis, and/or recurrence of one or more tumors.

A p53-deficient cancer cell may have a p53- deficient genotype or phenotype. For the purposes described herein a p53-deficient cancer cell has, e.g., a mutation within the p53 gene, e.g., insertion(s), deletion(s) of part or all of the gene, substitution(s) etc. such that no p53 or a mutant p53, e.g., one that does not bind DNA effectively, is produced.

Prostate apoptosis response-4 (PAR-4) is a pro-apoptotic gene identified in prostate cancer cells undergoing apoptosis. PAR-4 protein exists in the cytoplasm, endoplasmic reticulum, and nucleus. PAR-4 protein, which contains a leucine zipper domain at the carboxy-terminus, functions as a transcriptional repressor in the nucleus. In the nucleus, PAR-4 interacts with the transcription factor WT1 to inhibit the antiapoptotic protein Bcl2, and PAR-4 also inhibits the topoisomerase TOP1. In the cytoplasm, PAR-4 can be regulated by the kinases Akt and ζPKC and can inhibit the transcription factor NEκB to promote cell death. PAR-4 induces apoptosis in diverse cancer cells but not in normal cells. PAR-4 is ubiquitously expressed in normal cells and tissues. Intracellular PAR-4 is phosphorylated by protein kinase A (PKA) at a specific T155 residue found in the SAC (selective for apoptosis induction in cancer cells) domain of PAR-4. The phosphorylation of the Thr155 residue allows trafficking of the Fas/FasL to the plasma membrane. The Fas/FasL interacts with FADD causing activation of the Fas/FasL-FADD-Caspase 8 apoptotic death pathway. The basal level of PKA in normal cells is insufficient to cause T155 phosphorylation, thereby making normal cells resistant to PAR-4 mediated apoptosis.

Extracellular PAR-4 binds to its receptor GRP-78 on the cancer cell surface and induces apoptosis. In contrast, normal cells express low to undetectable levels of basal or inducible cell surface GRP-78 and are resistant to apoptosis by extracellular PAR-4. GRP-78, previously known as a prosurvival protein, is involved in PAR-4- and TRAIL induced apoptotic signaling. TRAIL binds cell surface receptors such as DR5 and DR4, which recruit FADD and caspase 8 in a DISC (death-inducing signaling complex) to initiate extrinsic cell death. Both intracellular and secreted PAR-4 play a role in apoptosis induction by caspase-dependent mechanisms.

Because the baseline levels of PAR-4 secreted by normal cells are generally inadequate to cause massive apoptosis in cancer cell cultures, secretogogues that bolster the release of PAR-4 constitute an important therapeutic advance. Nutlin-3a, originally developed as an MDM2 inhibitor, stimulated PAR-4 secretion at micromolar levels in mouse embryonic fibroblast (MEF) cells.

PAR-4 co-localized with vimentin. Vimentin is a type III intermediate filament (IF) protein that is expressed in mesenchymal cells and is the major cytoskeletal component of mesenchymal cells. Secretogogues like nutlin / arylquin 1 which cause stimulation of PAR-4 secretion exhibits this function by binding to vimentin and releasing vimentin-bound PAR-4 for secretion. This secreted PAR-4 binds to its receptor GRP-78, which is upregulated on the surface of cancer cells, and induces apoptosis. There are several non-FDA approved small molecules that can increase secretion of PAR-4 from normal cells in mice and the sera from these mice induced ex vivo apoptosis in cancer cell cultures.

Surprisingly, the PAR-4 inducing agent was found to promote secretion of PAR-4 both in-vitro and in-vivo.

Pyronaridine is described in Croft et al Malar. J, 2012, 11, 270.

Narasin and its biological activity is described in Miller et. al Biochem Pharmacol. 2010 May 1; 79(9): 1272-1280.

Adapalene and its biological activity is described in Piskin & Uzunali Therapeutics and Clinical Risk Management 2007:3(4) 621-624.

Mebendazole is described in United States patent no. 3,657,267 and Brugmans et al., J. Am. Med. Assoc. 217, 313 (1971).

Mefloquine and its preparation was first described by Ohnmacht et al. (J. Med. Chem. 1971, 14, 926) in 1971. A more detailed account of the stereochemistry, synthesis, and anti-malarial activity of the isomers of Mefloquine is given by Carroll and Blackwell (J. Med. Chem. 1974, 17, 210-219) in 1974.

Terconazole is described in United States patent no. 4,223,036.

Tafenoquine is described in United States patent no. 4,617,394.

Minoxidil is described in United States patent no. 3,382,247.

Nalidixic acid is described in United States patent no. 3,590,036.

Sparfloxacin is described in United States patent no. 4,795,751.

Pipemidic acid is described in United States patent no. 3,887,557.

Lopinavir is described in United States patent no. 5,914,332.

Ofloxacin is described in United States patent no. 4,382,892.

Also described herein is a method for treating a subject in need thereof with a PAR-4 inducing agent to increase apoptosis of cancer cells and to reduce the metastasis and proliferation and/or survival of cancer cells.

The term "PAR-4 inducing agent" is used in broad sense to include not only "PAR-4 inducing agent" per se but also its pharmaceutically acceptable derivatives thereof. Suitable pharmaceutically acceptable derivatives include pharmaceutically acceptable salts, pharmaceutically acceptable solvates, pharmaceutically acceptable hydrates, pharmaceutically acceptable anhydrates, pharmaceutically acceptable enantiomers, pharmaceutically acceptable esters, pharmaceutically acceptable isomers, pharmaceutically acceptable polymorphs, pharmaceutically acceptable prodrugs, pharmaceutically acceptable tautomers, pharmaceutically acceptable complexes etc. However, the "PAR-4 inducing agents" of the present invention are defined in claim 1.

Also disclosed is a method of killing cancer cells, e.g., by inducing apoptosis, or inhibiting cancer cell proliferation, cancer cell metastasis, and/or recurrence of tumors in a subject comprising administering an effective amount of an agent to a subject in need of treatment for sufficient time to increase prostate apoptosis responsive 4 (PAR-4) secretion from normal cells in the subject, wherein the agent is PAR-4 inducing agent, or pharmaceutically acceptable derivative thereof , and wherein the agent may or may not administered with a chemotherapeutic.

The term "chemotherapeutic" can be used synonymously with the "anticancer" and is used in broad sense to include not only DNA-interactive Agents, Antimetabolites, Tubulin-Interactive Agents, Hormonal agents and others such as Asparaginase or hydroxyurea.

The PAR-4 inducing agent for use according to the claims comprises contacting a population of cells comprising cancer cells and normal cells with an effective amount of the agent for a sufficient time comprises administering to a subject in need thereof in an effective amount, e.g., an amount sufficient to increase PAR-4 expression and/or secretion level above the level of PAR-4 expressed, secreted, or both by a cell prior to administration of the PAR-4 inducing agent by any suitable route of administration. In another aspect, an effective amount of PAR-4 inducing is an amount sufficient to increase apoptosis of cancer cells, and/or reduce proliferation and/or reduce metastasis of cancer cells in the subject. Preferably, the PAR-4 inducing agent is administered in an amount that induces apoptosis in cancer cells.

By way of non-limiting example, an effective amount of PAR-4 inducing agent contacted with cells may include for example about 10 nM to about 1000 µM, about 200 nM to about 100 µM, about 150 nM to about 75 µM, about 100 nM to about 50 µM, about 100 nM to about 40 µM, about 100 nM to about 35 µM, about 100 nM to about 30 µM, about 100 nM to about 25 µM.

In various embodiments, an effective amount of PAR-4 inducing agent is contacted with cells for at least about 2 hours, at least about 5 hours, at least about 10 hours, at least about 16 hours, at least about 18 hours, at least about 20 hours, at least about 24 hours, at least about 2 days, at least about 3 days, at least about 5 days, at least about a week, at least about 2 weeks, or more than 2 weeks.

By way of further non-limiting example, an effective amount of PAR-4 inducing agent administered to a subject in need thereof may include about 0.1-2000 mg daily, about 100- 1500 mg daily, about 150-1200 mg daily, about 200-1200 mg daily, about 100 mg daily, about 200 mg daily, about 300 mg daily, about 400 mg daily, about 500 mg daily, about 750 mg daily, about 1000 mg daily, about 1200 mg daily or more than about 1200 mg daily.

In various embodiments, an effective amount of PAR-4 inducing agent is administered to a subject in need thereof for 1 day, for 2 days, for 5 days, for about 1 week, for about 2 weeks, for at least about 1 month, for at least about 2 months, for at least about 3 months, for at least about 6 months, for at least about 8 months, for at least about 1 year, or for more than 1 year. It is understood that the duration of treatment will depend upon the stage of cancer and whether the cancer has gone into remission.

The actual amount encompassed by the term effective amount will depend on the route of administration, the type of subject being treated, and the physical characteristics of the specific subject under consideration, e.g., their age, weight, severity of disease, comorbidities. These factors and their relationship to determining this amount are well known to skilled practitioners in the medical, and other related arts and one of skill in the art can readily determine the appropriate effective dose of PAR-4 inducing agent to be administered to a subject to achieve the desired levels of PAR-4 secretion. Likewise, the dosing schedule may be readily determined by the skilled artisan.

Preferably, PAR-4 inducing agent may be administered to the subject by any means currently used in the art, e.g., orally, subcutaneously, transdermaly, intravenously, intramuscularly, parenterally, rectally, intranasally, intratumorally etc. For example, the PAR-4 inducing agent may be administered orally to the subject in any pharmaceutically acceptable form, e.g., in the form of a tablet, a capsule, a syrup, or an elixir, or infused or injected by, e.g., an intra-peritoneal or intravenous or intramuscular route, and in an amount sufficient to increase the levels of PAR-4 in the subject, preferably to a level that is sufficient to kill the cancer cells, e.g., induce apoptosis in cancer cells, and/or inhibit proliferation and/or inhibit metastasis. Pharmaceutically acceptable forms may also comprise one or more pharmaceutically acceptable carriers, diluents, or excipients. Pharmaceutically acceptable carriers, diluents, or excipients are known in the art such as those described in, for example, Remington's Pharmaceutical Sciences, Mack Publishing Co. (A. R. Gennaro edit. 1985).

Preferably, PAR-4 inducing agent may be provided in the form of a pharmaceutically composition such as but not limited to, unit dosage forms including tablets, capsules (filled with powders, pellets, beads, mini-tablets, pills, micro-pellets, small tablet units, multiple unit pellet systems (MUPS), disintegrating tablets, dispersible tablets, granules, and microspheres, multiparticulates), sachets (filled with powders, pellets, beads, mini-tablets, pills, micro-pellets, small tablet units, MUPS, disintegrating tablets, dispersible tablets, granules, and microspheres, multiparticulates), powders for reconstitution and sprinkles, however, other dosage forms such as controlled release formulations, lyophilized formulations, modified release formulations, delayed release formulations, extended release formulations, pulsatile release formulations, dual release formulations and the like. Liquid or semisolid dosage form (liquids, suspensions, solutions, dispersions, ointments, creams, emulsions, microemulsions, sprays, spot-on, and the like), injection preparations, parenteral, topical, inhalations, buccal, nasal etc. may also be envisaged under the ambit of the invention.

The inventors of the present invention have also found that the solubility properties of the PAR-4 inducing agent are improved by nano-sizing thus leading to better bioavailability and dose reduction of the drug.

In one embodiment, the PAR-4 inducing agent may be present in the form of nanoparticles which have an average particle size of less than 2000 nm.

Suitable excipients may be used for formulating the dosage forms according to the present invention such as, but not limited to, surface stabilizers or surfactants, viscosity modifying agents, polymers including extended release polymers, stabilizers, disintegrants or super disintegrants, diluents, plasticizers, binders, glidants, lubricants, sweeteners, flavoring agents, anti-caking agents, opacifiers, anti-microbial agents, antifoaming agents, emulsifiers, buffering agents, coloring agents, carriers, fillers, anti-adherents, solvents, taste-masking agents, preservatives, antioxidants, texture enhancers, surface stabilisers, channeling agents, coating agents or combinations thereof.

The cancer cells and cancers that are treated may be any type such as but not limiting to Sarcoma, Carcinoma, Leukemia, Germ Cell Tumor, Blastoma and Lymphoma and Myeloma and /or any combination thereof.

In an embodiment of the present invention, a cancer cell or tumor is located in a highly vascularized tissue. In the context of the present invention, a highly vascularized tissue includes any tissue with better than average vascularization. For example, without limitation, highly vascularized tissue includes blood, lung, liver, skin, or kidney.

A cancer cell or cancer that is located in a highly vascularized tissue may have originated in that or another highly vascularized tissue or may have travelled to a highly vascularized tissue from any other tissue by metastasis.

Preferably the PAR-4 inducing agent or pharmaceutically acceptable derivative thereof, may or may not co-administered with a chemotherapeutic or other anticancer drugs.

The agents for use according to the present invention may also include treatment with PAR-4 inducing agent and an effective amount of ionizing radiation. Ionizing radiation has been used in the treatment of a variety of cancers, including melanoma (Khan et al. Onco Targets Ther. 2011; 4: 137-148). An effective amount of ionizing radiation may include any amount that is sufficient to kill cancer cells, inhibit cancer cell proliferation, cancer cell metastasis, and/or recurrence of one or more tumors. In an embodiment, an effective amount is about 1-5 Gy of radiation, 2-4 Gy of radiation, or about 2 Gy, about 3 Gy, about 4 Gy, or about 5 Gy of radiation. An effective amount of ionizing radiation will be apparent to the skilled artisan, and in a preferred embodiment, is less than the amount of ionizing radiation required to kill cancer cells, inhibit cancer cell proliferation, cancer cell metastasis, and/or recurrence of one or more tumors in the absence of treatment with PAR-4 inducing agent.

An effective amount of PAR-4 inducing agent may be co-administered with the ionizing radiation treatment. Improved effectiveness of the radiation therapy with administration of PAR-4 inducing agent may be manifested as an increase in cancer cell killing, e.g., cancer cell apoptosis, inhibited proliferation of cancer cells, or inhibited metastasis of cancer cells, as compared to similar subjects in need thereof treated with the same amount of ionizing radiation but without PAR-4 inducing agent treatment. The improved effectiveness of the radiation therapy with administration of PAR-4 inducing agent may alternatively or additionally be manifested by achieving cancer cell killing, e.g., cancer cell apoptosis, inhibited proliferation of cancer cells, or inhibited metastasis of cancer cells, with reduced amounts of ionizing radiation than are historically required to achieve such anticancer effects. The effects of PAR-4 inducing agent treatment and ionizing radiation can be unexpectedly more than additive. A subject in need of treatment is a subject who has cancer, a subject who has been treated for a cancer and is in remission or cancer free, or a subject having a recurrent cancer. The cancer may be any cancer, including but not limited to, e.g., prostate, breast, skin (e.g., melanoma), and/or lung cancer, or any cancer disclosed herein. The cancer may be an androgen independent prostate cancer, a breast cancer that expresses no or low levels of PAR-4, a breast cancer that is highly aggressive, estrogen receptor-negative, high-grade (grade 3) or basal-like tumor, or a non-small cell lung cancer, a small cell lung cancer, or a lung carcinoid tumor or brain tumors. The cancer may be a melanoma, e.g., superficial spreading melanoma, nodular melanoma, lentigno maligna melanoma, or desmoplastic melanoma. A subject in need of treatment may also include a subject in need of prophylactic treatment, where a subject in need of prophylactic treatment would be, e.g., a subject who does not currently have a cancer but has been determined to be at higher risk of developing a cancer, particularly a prostate, breast, skin, or lung cancer or any cancer described herein, as compared with the risk of the general population of developing that cancer.

Also disclosed are methods for reducing the recurrence of a cancer comprising administering PAR-4 inducing agent, or pharmaceutically acceptable derivative thereof , with or without a chemotherapeutic agent, to a subject in need thereof in an amount sufficient to reduce the recurrence of a cancer in the subject. In the methods described herein, the PAR-4 inducing agent or pharmaceutically acceptable derivative thereof may be administered to the subject in need thereof in combination with another agent that increases the production and/or secretion of PAR-4.

Also disclosed is a method for prophylactically reducing the risk of a subject developing cancer comprising administering PAR-4 inducing agent, or pharmaceutically acceptable derivative thereof to a subject in need thereof to elevate PAR-4 expression from normal cells to a level that enhances apoptosis of cancer cells.

It is understood that the methods disclosed herein may include a step of selecting a subject in need thereof, by identifying a subject who has a cancer(s) described herein, or who has been treated for such cancer(s) and is in remission or cancer free, or has a recurrent cancer(s), or is in need of prophylactic treatment, and then administering an effective amount of PAR-4 inducing agent with or without an effective amount other anticancer agent, or ionizing radiation, or combinations thereof, to such subject as described herein.

The following models and examples are for the purpose of illustration of the invention only and are not intended in any way to limit the scope of the present invention.

### Example 1. In-vitro PAR-4 induction by Pyronaridine

### Objective:

The objective of the current study was to check the ability of Pyronaridine to induce PAR-4 secretion from mouse embryonic fibroblast cells.

### Methodology:

Mouse embryonic fibroblast cells were harvested from exponential phase cultures. After a 24 h recovery period to allow the cells to resume exponential growth the cells were treated with 25 µM of Pyronaridine followed by further 18-21 h of incubation. Induction of PAR-4 was checked in the cell supernatant and cell lysate by subjecting the samples to Western blot analysis with antibodies specific for PAR-4 and actin. The samples were also subjected to SDS/PAGE and Coomassie blue staining to determine albumin levels in serum from the Conditioned Medium (CM) as another loading control.

### Results:

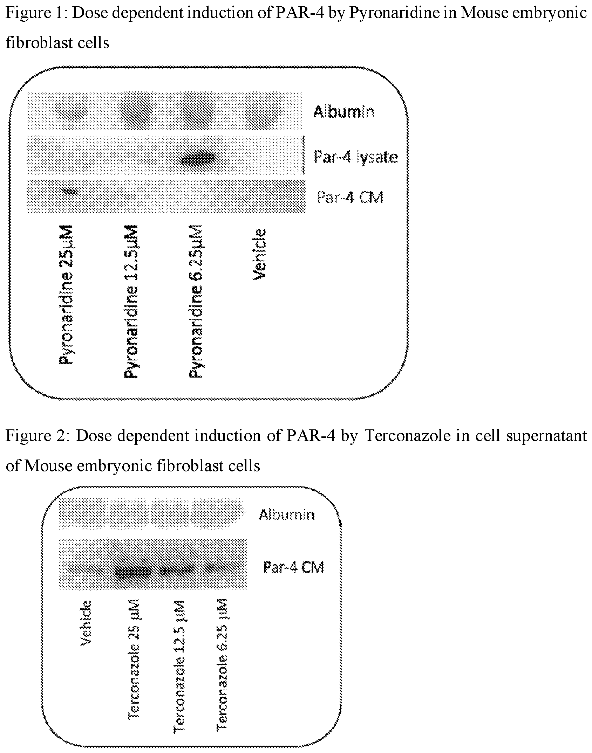

Pyronaridine was able to induce robust PAR-4 secretion in the cell supernatant from normal mouse embryonic fibroblast cells in a dose dependent manner. The results are depicted in Figure 1.

### Example 2. In-vitro PAR-4 induction by Terconazole:

### Objective:

The objective of the current study was to check the ability of Terconazole to induce PAR-4 secretion from mouse embryonic fibroblast cells.

### Methodology:

Mouse embryonic fibroblast cells were harvested from exponential phase cultures. After a 24 h recovery period to allow the cells to resume exponential growth the cells were treated with 25 µM of Terconazole followed by further 18-21 h of incubation. Induction of PAR-4 was checked in the cell supernatant and cell lysate by subjecting the samples to Western blot analysis with antibodies specific for PAR-4 and actin. The samples were also subjected to SDS/PAGE and Coomassie blue staining to determine albumin levels in serum from the CM as another loading control.

### Results:

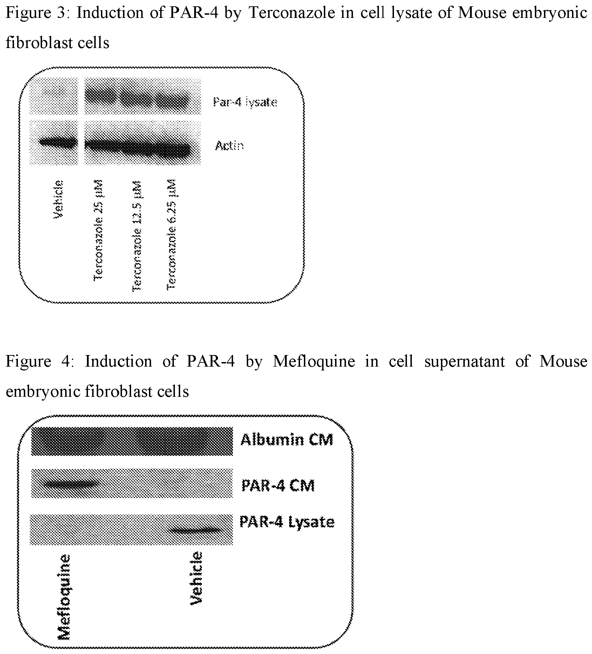

Terconazole was able to induce robust PAR-4 secretion in the cell supernatant from normal mouse embryonic fibroblast cells in a dose dependent manner. The results are depicted in Figure 2 and Figure 3.

### Example 3. In-vitro PAR-4 induction by Mefloquine

### Objective:

The objective of the current study was to check the ability of Mefloquine to induce PAR-4 secretion from mouse embryonic fibroblast cells

### Methodology:

Mouse embryonic fibroblast were harvested from exponential phase cultures. After a 24 h recovery period to allow the cells to resume exponential growth the cells were treated with 25 µM of Meloquine followed by further 18-21 h of incubation.

Induction of PAR-4 was checked in the cell supernatant and cell lysate by subjecting the samples to Western blot analysis with antibodies specific for PAR-4 and actin. The samples were also subjected to SDS/PAGE and Coomassie blue staining to determine albumin levels in serum from the cell supernatant as another loading control. Dose dependent induction of PAR-4 by Mefloquine was also checked in Mouse embryonic fibroblast cells.

### Results:

Mefloquine was able to induce robust PAR-4 secretion in the cell supernatant from normal mouse embryonic fibroblast cells. The results are depicted in Figure 4.

### Example 4. In-vivo PAR-4 induction by Mefloquine

### Objective:

The objective of the current study was to check the ability of Mefloquine to induce PAR-4 secretion systemically in C57BL6 mice.

### Methodology:

Immunocompetent mice were orally administered with 10 mg/kg and 51.2 mg/kg body weight of Mefloquine or vehicle for 3 consecutive days followed by collection of blood 24 hrs later, serum separation and testing for systemic levels of PAR-4.

### Results:

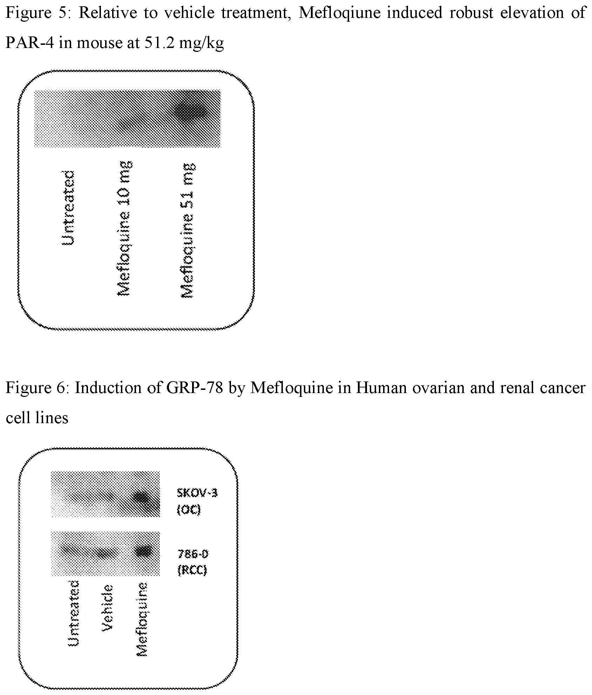

Relative to vehicle treatment, Mefloqiune induced robust elevation of PAR-4 in mouse serum and 51.2 mg/kg. The results are depicted in Figure 5.

### Example 5. In-vitro GRP-78 induction by Mefloquine

### Objective

The objective of the current study was to check the ability of Mefloquine to induce GRP-78 expression in Human ovarian cancer and renal cancer cell lines.

### Methodology

Human ovarian cancer cell line SKOV-3 and renal cancer cell line 786-O were harvested from exponential phase cultures. After a 24 h recovery period to allow the cells to resume exponential growth the cells were treated with 25 µM of Mefloquine followed by further 18-21 h of incubation. Induction of GRP-78 expression was checked in the cell lysate by subjecting the samples to Western blot analysis with antibodies specific for GRP-78.

### Results

Mefloquine induced GRP-78 expression in both SKOV-3 and 786-O at the test concentration of 25 µM. The results are depicted in Figure 6.

### Example 6. In-vitro PAR-4 induction by Narasin

### Objective:

The objective of the current study was to check the ability of Narasin to induce PAR-4 secretion from mouse embryonic fibroblast cells.

### Methodology:

Mouse embryonic fibroblast cells were harvested from exponential phase cultures. After a 24 h recovery period to allow the cells to resume exponential growth the cells were treated with 25 µM of Narasin followed by further 18-21 h of incubation. Induction of PAR-4 was checked in the cell supernatant and cell lysate by subjecting the samples to Western blot analysis with antibodies specific for PAR-4 and actin. The samples were also subjected to SDS/PAGE and Coomassie blue staining to determine albumin levels in serum from the CM as another loading control.

### Results:

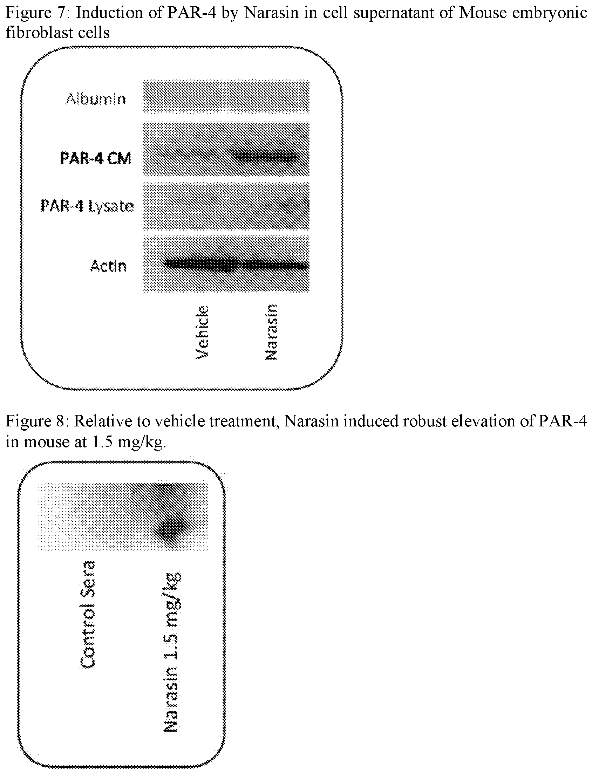

Narasin was able to induce robust PAR-4 secretion in the cell supernatant from normal mouse embryonic fibroblast cells. The results are depicted in Figure 7.

### Example 7. In-vivo PAR-4 induction by Narasin

### Objective:

The objective of the current study was to check the ability of Narasin to induce PAR-4 secretion systemically in C57BL6 mice.

### Methodology:

Immunocompetent mice were orally administered with 1.5 mg/kg body weight of Narasin or vehicle for 3 consecutive days followed by collection of blood 24 hrs later, serum separation and testing for systemic levels of PAR-4.

### Results:

Relative to vehicle treatment, Narasin induced robust elevation of PAR-4 in mouse serum. The results are depicted in Figure 8.

### Example 8. In-vitro PAR-4 induction by Mebendazole

### Objective:

The objective of the current study was to check the ability of Mebendazole to induce PAR-4 secretion from mouse embryonic fibroblast cells

### Methodology:

Mouse embryonic fibroblast were harvested from exponential phase cultures. After a 24 h recovery period to allow the cells to resume exponential growth the cells were treated with 25 µM of Mebendazole followed by further 18-21 h of incubation. Induction of PAR-4 was checked in the cell supernatant and cell lysate by subjecting the samples to Western blot analysis with antibodies specific for PAR-4 and actin. The samples were also subjected to SDS/PAGE and Coomassie blue staining to determine albumin levels in serum from the cell supernatant as another loading control. Dose dependent induction of PAR-4 by Mebendazole was also checked in Mouse embryonic fibroblast cells.

### Results:

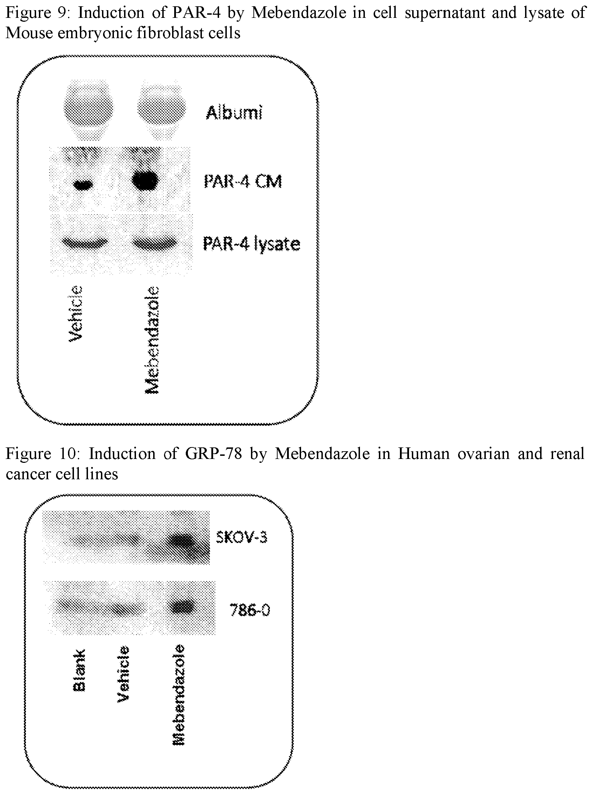

Mebendazole was able to induce robust PAR-4 secretion in the cell supernatant from normal mouse embryonic fibroblast cells. The results are depicted in Figure 9.

### Example 9. In-vitro GRP-78 induction by Mebendazole

### Objective:

The objective of the current study was to check the ability of Mebendazole to induce GRP-78 expression in Human ovarian cancer and renal cancer cell lines.

### Methodology:

Human ovarian cancer cell line SKOV-3 and renal cancer cell line 786-O were harvested from exponential phase cultures. After a 24 h recovery period to allow the cells to resume exponential growth the cells were treated with 25 µM of Mebendazole followed by further 18-21 h of incubation. Induction of GRP-78 expression was checked in the cell lysate by subjecting the samples to Western blot analysis with antibodies specific for GRP-78.

### Results:

Mebendazole induced GRP-78 expression in both SKOV-3 and 786-O at the test concentration of 25 µM. The results are depicted in Figure 10.

### Example 10. In-vitro PAR-4 induction by Tefenoquine

### Objective

The objective of the current study was to check the ability of Tafenoquine to induce PAR-4 secretion from mouse embryonic fibroblast cells

### Methodology:

Mouse embryonic fibroblast were harvested from exponential phase cultures. After a 24 h recovery period to allow the cells to resume exponential growth the cells were treated with 25 µM of Tafenoquine followed by further 18-21 h of incubation. Induction of PAR-4 was checked in the cell supernatant and cell lysate by subjecting the samples to Western blot analysis with antibodies specific for PAR-4 and actin. The samples were also subjected to SDS/PAGE and Coomassie blue staining to determine albumin levels in serum from the cell supernatant as another loading control. Dose dependent induction of PAR-4 by Tafenoquine was also checked in Mouse embryonic fibroblast cells.

### Results:

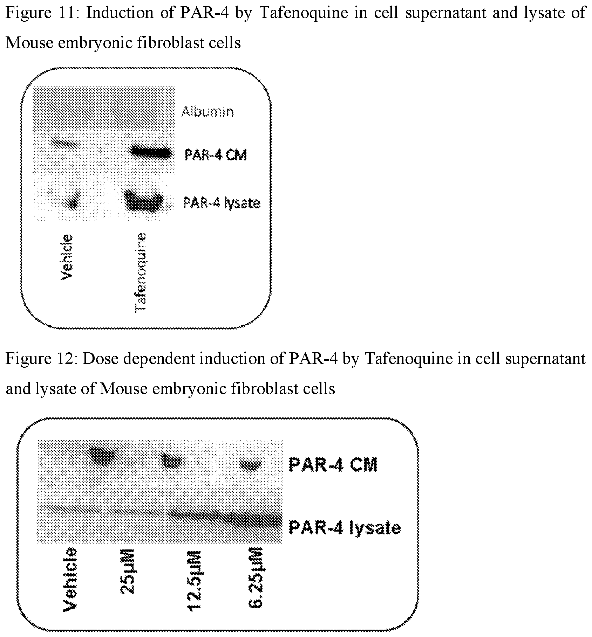

Tafenoquine was able to induce robust PAR-4 secretion in the cell supernatant from normal mouse embryonic fibroblast cells. The results are depicted in Figure 11 & 12

### Example 11. In-vitro GRP-78 induction by Tefenoquine

### Objective

The objective of the current study was to check the ability of Tafenoquine to induce GRP-78 expression in Human ovarian cancer and renal cancer cell lines.

### Methodology

Human ovarian cancer cell line SKOV-3 and renal cancer cell line 786-O were harvested from exponential phase cultures. After a 24 h recovery period to allow the cells to resume exponential growth the cells were treated with 25 µM of Tafenoquine followed by further
18-21 h of incubation. Induction of GRP-78 expression was checked in the cell lysate by subjecting the samples to Western blot analysis with antibodies specific for GRP-78.

### Results

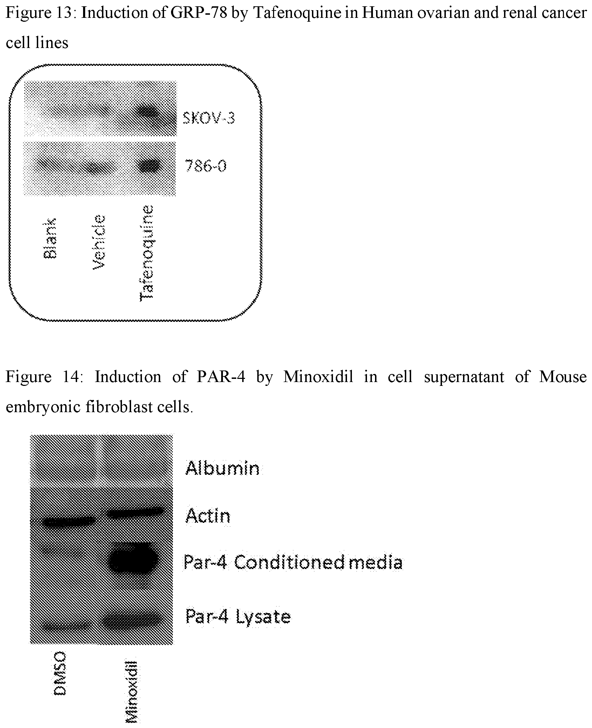

Tafenoquine induced GRP-78 expression in both SKOV-3 and 786-O at the test concentration of 25 µM. The results are depicted in Figure 13

### Example 12. In-vitro PAR-4 induction by Minoxidil

### Objective:

The objective of the current study was to check the ability of Minoxidil to induce PAR-4 secretion from mouse embryonic fibroblast cells.

Methodology: The experiment was conducted as per method set in an Example 1.

### Results:

Minoxidil was able to induce robust PAR-4 secretion in the cell supernatant from normal mouse embryonic fibroblast cells. The results are depicted in Figure 14.

### Example 13. In-vitro PAR-4 induction by Nalidixic acid

### Objective:

The objective of the current study was to check the ability of nalidixic acid to induce PAR-4 secretion from mouse embryonic fibroblast cells.

Methodology: The experiment was conducted as per method set in an Example 1.

### Results

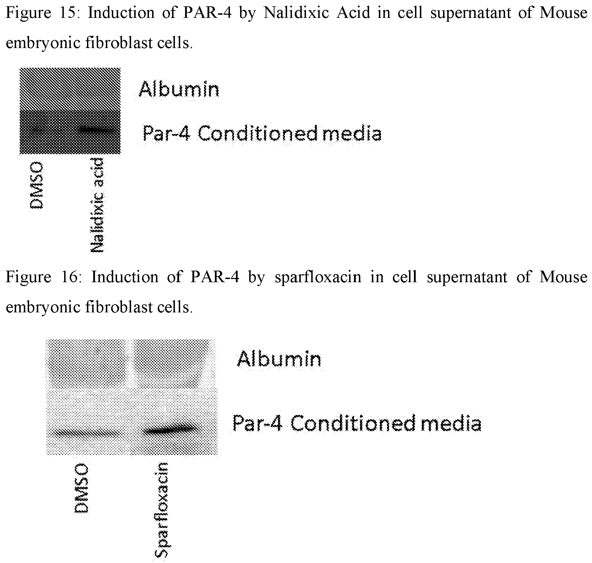

Nalidixic Acid was able to induce robust PAR-4 secretion in the cell supernatant from normal mouse embryonic fibroblast cells. The results are depicted in Figure 15

### Example 14. In-vitro PAR-4 induction by Sparfloxacin

### Objective:

The objective of the current study was to check the ability of sparfloxacin to induce PAR-4 secretion from mouse embryonic fibroblast cells.

Methodology: The experiment was conducted as per method set in an Example 1.

### Results

Sparfloxacin was able to induce robust PAR-4 secretion in the cell supernatant from normal mouse embryonic fibroblast cells. The results are depicted in Figure 16.

### Example 15. In-vitro PAR-4 induction by Pipemidic Acid

### Objective:

The objective of the current study was to check the ability of pipemidic acid to induce PAR-4 secretion from mouse embryonic fibroblast cells.

Methodology: The experiment was conducted as per method set in an Example 1.

### Results

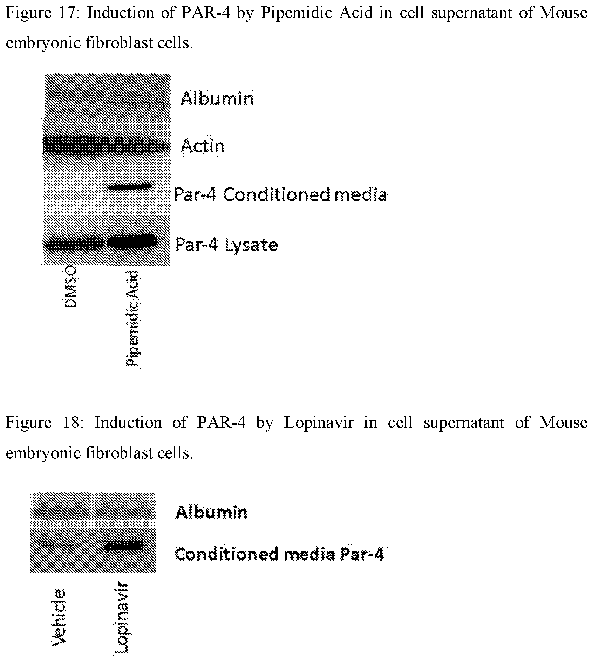

Pipemidic Acid was able to induce robust PAR-4 secretion in the cell supernatant from normal mouse embryonic fibroblast cells. The results are depicted in Figure 17.

### Example 16. In-vitro PAR-4 induction by Lopinavir

### Objective:

The objective of the current study was to check the ability of Lopinavir to induce PAR-4 secretion from mouse embryonic fibroblast cells.

Methodology: The experiment was conducted as per method set in an Example 1.

### Results

Lopinavir was able to induce robust PAR-4 secretion in the cell supernatant from normal mouse embryonic fibroblast cells. The results are depicted in Figure 18.

### Example 17. In-vitro PAR-4 induction by Ofloxacin

### Objective:

The objective of the current study was to check the ability of ofloxacin to induce PAR-4 secretion from mouse embryonic fibroblast cells.

Methodology: The experiment was conducted as per method set in an Example 1.

### Results

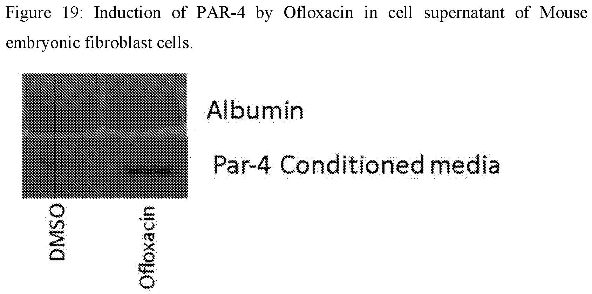

Ofloxacin was able to induce robust PAR-4 secretion in the cell supernatant from normal mouse embryonic fibroblast cells. The results are depicted in Figure 19.

## Claims

1. An agent for use in the treatment of cancer in a subject in need thereof by contacting a population of cells comprising cancer cells and normal cells with an effective amount of the agent which is selected from a group consisting of: mebendazole, pyronaridine, tafenoquine, minoxidil, nalidixic acid, sparfloxacin, pipemidic acid, and ofloxacin..

2. The agent for use according to claim 1, wherein the effective amount of agent is in the range of from 10nM to 1000µM.

3. The agent for use according to claim 2, wherein the effective amount of agent is in the range of from 100nM to 25µM.

4. The agent for use according to claim 1, wherein the effective amount of agent is in the range of from 0.1mg to 2000 mg daily.

5. The agent for use according to claim 1, wherein the agent is contacted with the population of cells comprising cancer cells and normal cells for at least about 2 hours.

6. The agent for use according to claim 1, wherein the cancer cell is p53 deficient.

7. The agent for use according to claim 1, wherein the agent is in the form of tablet, capsule, syrup, elixir, infusion or injection.

8. The agent for use according to claim 1, wherein the agent is in the form of nanoparticles of average particle size of less than 2000nm.

9. The agent for use according to claim 1, wherein the cancer cells are sarcoma, carcinoma, leukemia, germ cell tumor, blastoma, lymphoma, myeloma cancer cell or any combination thereof.

10. The agent for use according to claim 1, wherein the cancer cell is located in a vascularized tissue.

11. The agent for use according to claim 1, wherein the agent is administered in combination with at least one additional cancer therapy.

12. The agent for use according to claim 11, wherein the at least one additional cancer therapy comprises:
(i) administration of a chemotherapeutic agent;
(ii) ionizing radiation; or
(iii) administration of an additional PAR-4 inducing agent.

13. The agent for use according to claim 1, wherein the cancer is prostate cancer, breast cancer, skin cancer, lung cancer or any combination thereof.

## Patentansprüche

1. Mittel zur Verwendung bei der Behandlung von Krebs bei einem Individuum, das dessen bedarf, durch Inkontaktbringen einer Population von Zellen,
umfassend Krebszellen und normale Zellen, mit einer wirksamen Menge des Mittels, das ausgewählt ist aus einer Gruppe bestehend aus: Mebendazol, Pyronaridin, Tafenoquin, Minoxidil, Nalidixinsäure, Sparfloxacin, Pipemidsäure und Ofloxacin.

2. Mittel zur Verwendung nach Anspruch 1, wobei die wirksame Menge des Mittels im Bereich von 10 nm bis 1000 pm liegt.

3. Mittel zur Verwendung nach Anspruch 2, wobei die wirksame Menge des Mittels im Bereich von 100 nm bis 25 pm liegt.

4. Mittel zur Verwendung nach Anspruch 1, wobei die wirksame Menge des Mittels im Bereich von 0,1 mg bis 2000 mg täglich liegt.

5. Mittel zur Verwendung nach Anspruch 1, wobei das Mittel mit der Population von Zellen, umfassend Krebszellen und normale Zellen, für mindestens etwa 2 Stunden in Kontakt gebracht wird.

6. Mittel zur Verwendung nach Anspruch 1, wobei die Krebszelle p53-defizient ist.

7. Mittel zur Verwendung nach Anspruch 1, wobei das Mittel in Form einer Tablette, einer Kapsel, eines Sirups, eines Elixiers, einer Infusion oder einer Injektion vorliegt.

8. Mittel zur Verwendung nach Anspruch 1, wobei das Mittel in Form von Nanoteilchen mit einer durchschnittlichen Teilchengröße von weniger als 2000 nm vorliegt.

9. Mittel zur Verwendung nach Anspruch 1, wobei die Krebszellen Sarkom, Karzinom, Leukämie, Keimzelltumor, Blastom, Lymphom, Myelomkrebszellen oder eine Kombination davon sind.

10. Mittel zur Verwendung nach Anspruch 1, wobei sich die Krebszelle in einem vaskularisierten Gewebe befindet.

11. Mittel zur Verwendung nach Anspruch 1, wobei das Mittel in Kombination mit mindestens einer zusätzlichen Krebstherapie verabreicht wird.

12. Mittel zur Verwendung nach Anspruch 11, wobei die mindestens eine zusätzliche Krebstherapie Folgendes umfasst:
(i) Verabreichung eines chemotherapeutischen Mittels;
(ii) Ionenstrahlung; oder
(iii) Verabreichung eines zusätzlichen PAR-4-induzierenden Mittels.

13. Mittel zur Verwendung nach Anspruch 1, wobei der Krebs Prostatakrebs, Brustkrebs, Hautkrebs, Lungenkrebs oder eine beliebige Kombination davon ist.

## Revendications

1. Agent destiné à être utilisé dans le traitement du cancer chez un sujet nécessitant un tel traitement, par contact avec une population de cellules
comprenant des cellules cancéreuses et des cellules normales avec une quantité efficace de l'agent qui est choisi dans un groupe constitué de : mébendazole, pyronaridine, tafénoquine, minoxidil, acide nalidixique, sparfloxacine, acide pipémidique et ofloxacine.

2. Agent destiné à être utilisé selon la revendication 1, dans lequel la quantité efficace d'agent est dans la plage de 10 nM à 1000∘µM.

3. Agent destiné à être utilisé selon la revendication 2, dans lequel la quantité efficace d'agent est dans la plage de 100 nM à 25∘µM.

4. Agent destiné à être utilisé selon la revendication 1, dans lequel la quantité efficace d'agent est dans la plage de 0,1 mg à 2000 mg par jour.

5. Agent destiné à être utilisé selon la revendication 1, dans lequel l'agent est mis en contact avec la population de cellules comprenant des cellules cancéreuses et des cellules normales pendant au moins environ 2∘heures.

6. Agent destiné à être utilisé selon la revendication 1, dans lequel la cellule cancéreuse est déficiente en p53.

7. Agent destiné à être utilisé selon la revendication 1, dans lequel l'agent est sous la forme d'un comprimé, d'une capsule, d'un sirop, d'un élixir, d'une perfusion ou d'une injection.

8. Agent destiné à être utilisé selon la revendication 1, dans lequel l'agent est sous la forme de nanoparticules ayant une taille moyenne de particules inférieure à 2000 nm.

9. Agent destiné à être utilisé selon la revendication 1, dans lequel les cellules cancéreuses sont un sarcome, un carcinome, une leucémie, une tumeur de cellules germinales, un blastome, un lymphome, une cellule de cancer du myélome ou une combinaison quelconque de ceux-ci.

10. Agent destiné à être utilisé selon la revendication 1, dans lequel la cellule cancéreuse est située dans un tissu vascularisé.

11. Agent destiné à être utilisé selon la revendication 1, dans lequel l'agent est administré en combinaison avec au moins une cancérothérapie supplémentaire.

12. Agent destiné à être utilisé selon la revendication 11, dans lequel l'au moins une cancérothérapie supplémentaire comprend :
(i) l'administration d'un agent chimiothérapeutique ;
(ii) le rayonnement ionisant ; ou
(iii) l'administration d'un agent inducteur PAR-4 supplémentaire.

13. Agent destiné à être utilisé selon la revendication 1, dans lequel le cancer est le cancer de la prostate, le cancer du sein, le cancer de la peau, le cancer du poumon ou toute combinaison de ceux-ci.
